Europäisches Patentamt

European Patent Office

Office européen des brevets

(11) Numéro de publication: **0 546 899 B1**

# FASCICULE DE BREVET EUROPEEN

(45) Date de publication de fascicule du brevet: **02.08.95**

(51) Int. Cl.[6]: **G01B 11/16**, G01D 5/26

(21) Numéro de dépôt: **92403281.6**

(22) Date de dépôt: **04.12.92**

(54) **Structure à contrôle d'endommagement intrinsèque, procédés de fabrication et méthode d'utilisation.**

(30) Priorité: **11.12.91 FR 9115347**

(43) Date de publication de la demande:
**16.06.93 Bulletin 93/24**

(45) Mention de la délivrance du brevet:
**02.08.95 Bulletin 95/31**

(84) Etats contractants désignés:
**DE GB IT**

(56) Documents cités:
**EP-A- 0 158 116**
**EP-A- 0 162 303**
**WO-A-86/01286**
**DE-A- 3 419 580**

**APPLIED OPTICS, vol. 29, no. 15, 20 mai 1990, New York, US, pages 2223-2230, K. OKA ET AL: "Spectral and dynamic characteristics for excited and coupled modes in an externally perturbed birefringent single-mode fiber"**

(73) Titulaire: **THOMSON-CSF**
**173, Boulevard Haussmann**
**F-75008 Paris (FR)**

(72) Inventeur: **Bonniau, Philippe**
**Thomson-CSF,**
**SCPI,**
**50, rue J.P. Timbaud**
**F-92402 Courbevoie Cedex (FR)**
Inventeur: **Chazelas, Jean**
**Thomson-CSF,**
**SCPI,**
**50, rue J.P. Timbaud**
**F-92402 Courbevoie Cedex (FR)**
Inventeur: **Estang, Bernard**
**Thomson-CSF,**
**SCPI,**
**50, rue J.P. Timbaud**
**F-92402 Courbevoie Cedex (FR)**
Inventeur: **Lecuellet, Jérôme**
**Thomson-CSF,**
**SCPI,**
**50, rue J.P. Timbaud**
**F-92402 Courbevoie Cedex (FR)**
Inventeur: **Perrier, Bernard**
**Thomson-CSF,**
**SCPI,**
**50, rue J.P. Timbaud**
**F-92402 Courbevoie Cedex (FR)**

Rank Xerox (UK) Business Services
(3. 10/3.0 9/3.3.3)

(74) Mandataire: **Benoit, Monique et al**
**THOMSON-CSF**
**SCPI**
**B.P. 329**
**50, rue Jean-Pierre Timbaud**
**F-92402 Courbevoie Cédex (FR)**

## Description

L'invention se situe dans le domaine des structures intégrant un matériau composite, le matériau comportant des fibres optiques destinées à contrôler la fabrication et l'état en cours d'usage des parties de structures en matériau composite, en particulier des matériaux multicouches, elle concerne le matériau composite inclus dans la structure, un procédé de fabrication et une méthode de mesure utilisant la fibre pour la localisation et la détermination de la nature de défauts éventuels.

On sait qu'un matériau composite est constitué de fibres assemblées en différentes nappes formant des plis du matériau.

Selon les sollicitations mécaniques auxquelles va être soumis le matériau, les nappes peuvent être disposées selon différentes directions. Le matériau composite est ainsi composé de différentes couches ou plis. Il peut exister entre deux plis et ceci malgré les précautions prises lors de la fabrication, des défauts de fabrication. De tels défauts appelés délaminage peuvent également apparaître après fabrication par vieillissement, par suite des sollicitations en déformation provoquées par des vibrations ou des cycles de température. Ces défauts peuvent également apparaître par suite d'impact. Les délaminages quelle que soit leur origine ne provoquent aucun dommage directement visible. Or, ce phénomène par la possibilité de flambage local qu'il génère, réduit considérablement la résistance de la structure en compression. En dehors de cet aspect mécanique, l'hétérogénéité ainsi induite dans le matériau peut modifier d'autres caractéristiques. Ainsi, si le composite est utilisé comme radôme d'une antenne, le diagramme de rayonnement de cette dernière peut se trouver modifié.

Il est donc nécessaire de vérifier lors de la fabrication et ensuite de façon régulière que les défauts présents dans le matériau ne sont pas supérieurs à un seuil préalablement fixé.

Pour cela, des techniques de contrôle non destructif de structures mécaniques sont employées. Les techniques généralement employées font appel à des techniques d'interrogation extrinsèques, c'est le cas de la thermographie, de l'émission acoustique, des ultrasons, de la radiographie.

Toutes ces techniques de contrôle par des moyens extrinsèques doivent être effectuées en laboratoires après démontage de la structure contrôlée. Outre l'inconvénient du démontage et de l'immobilisation, les techniques employées nécessitent un long apprentissage pour connaître la nature des défauts, leurs dimensions et leurs localisations en fonction des résultats de mesure obtenus.

Compte tenu des progrès réalisés dans la fabrication de capteurs intrinsèques à fibres optiques, on a pensé à utiliser de tels capteurs en les incluant directement à l'intérieur du matériau composite.

Une telle inclusion est rapportée par le journal "Electronique international hebdo" n° 18 du 9 Mai 1991 page 15, dans un article intitulé "Améliorer les performances des composites par fibre optique". Cet article rapporte que le projet envisagé "consiste à noyer dans un composite une fibre optique continue à maintien de polarisation jouant le rôle de réseau de capteurs. C'est-à-dire où le paramètre à mesurer agit directement sur la fibre (à l'opposé des capteurs à fibre optique extrinsèques, où la fibre optique sert uniquement de support pour le transport d'information). Ici, la fibre optique modifie la nature du signal qui la parcourt en fonction des déformations et de la température. Selon le chef de file du projet, avec un pas entre chaque point de mesure de 10 cm, le nombre de points peut varier de 20 à 100 suivant la longueur de la fibre. Les premières applications visées sont dans le secteur de l'aéronautique. Mais Bertin, qui pour le traitement des mesures utilise le principe du codage en modulation spectrale, compte étendre le champ d'application du réseau de capteurs intrinsèques, notamment à la mesure de vibration d'une structure".

L'inconvénient principal de la méthode de mesure rapportée ci-dessus provient du nombre de pas qui est au maximum égal au quotient de la largeur du spectre total par la largeur du spectre le plus petit que l'on sait isoler. La méthode nécessite que le maillage de la pièce composite par les différents capteurs ait été prédéterminé. Il convient également de pouvoir séparer par l'analyse du signal capté en sortie de fibre, les modifications dues à la température et celles dues aux contraintes mécaniques.

Le problème technique résolu par la présente invention est donc de savoir quelle fibre optique à maintien de polarisation employer, comment la disposer à l'intérieur du matériau composite, la remplacer en cas d'endommagement et réaliser le matériau sans endommager la fibre optique et enfin, comment réaliser une mesure et l'exploiter pour savoir dans quelle zone se situe un défaut éventuel.

L'invention a donc pour but de permettre la détection et la localisation d'une irrégularité, que cette irrégularité soit due à un défaut de fabrication ou à un choc, un délaminage, à l'intérieur d'une pièce réalisée en matériau composite, la détection et la localisation étant réalisées par l'analyse d'un signal circulant dans la fibre, sans que le maillage le long de la fibre soit prédéterminé par la nature du signal qui y est injecté.

Le but est aussi de détecter des contraintes ou des ruptures de contraintes situées à l'intérieur d'une gamme prédéterminée. Il est enfin possible de pouvoir réaliser ces détections et localisations d'irrégularités en temps réel ou en temps différé. L'analyse en temps réel est particulièrement intéressante lorsque la pièce composite est le radôme d'une antenne dont il est important de s'assurer que le diagramme de rayonnement reste constant, par exemple un radôme d'antenne pour dispositif d'atterrissage aux instruments (I L S). Dans tous les cas, l'invention permet la détection de défauts sans démontage de la pièce, au moyen d'un seul instrument, par exemple un interféromètre de Michelson à miroir mobile.

A toutes ces fins l'invention a pour objet une structure comportant au moins partiellement un matériau composite, le matériau étant réalisé au moyen de nappes de fibres noyées dans une matrice, les différentes nappes formant des plis du matériau, le matériau comportant des moyens intrinsèques de détection et de localisation d'une irrégularité mécanique, ces moyens comportant au moins une fibre optique biréfringente à maintien de polarisation ayant un axe dit lent et un axe rapide, noyée dans la matrice et décrivant à l'intérieur du matériau un itinéraire connu caractérisé en ce que la fibre optique est incluse à l'intérieur d'un tubage de diamètre intérieur supérieur au diamètre extérieur de la fibre.

L'inclusion de la fibre, éventuellement revêtue d'une surcouche destinée à modifier la valeur d'un couplage entre les axes de propagation de la fibre, à l'intérieur d'un tubage, permet d'obtenir de façon bien plus aisée une plage de valeurs de contraintes, correspondant à une modification linéaire en fonction de la contrainte, d'un paramètre d'une radiation lumineuse se propageant dans la fibre.

Des modes de réalisation de matériaux composites incluant le capteur et son tubage ainsi que des courbes illustrant les résultats obtenus seront maintenant décrits, en référence aux dessins annexés dans lesquels :

- la figure 1, représente une section transversale d'une fibre utilisée;
- la figure 2, représente une coupe transversale d'un matériau composite incluant une fibre selon l'invention, la fibre étant représentée en coupe longitudinale;
- la figure 3, représente une demi-coupe transversale d'un matériau composite sandwich incluant une fibre selon l'invention, la fibre étant représentée en coupe longitudinale;
- les figures 4a, 4b, 4c représentent une coupe transversale d'un matériau composite incluant une fibre selon l'invention, la fibre étant placée sous des contraintes variables;

- la figure 5, est une courbe montrant la variation de l'intensité d'un signal optique en fonction de la contrainte subie localement par la fibre;
- la figure 6, est un schéma destiné à illustrer le fonctionnement de la fibre à maintien de polarisation;
- la figure 7, est un schéma illustrant le principe de la mesure;
- la figure 8, illustre un procédé de fabrication d'un matériau inclus dans une structure selon l'invention;
- la figure 9, est un diagramme température/pression utilisées dans un mode de fabrication de l'invention.

La fibre optique retenue pour ce genre d'application est la fibre FASE. Elle est décrite dans le brevet FR n° 8915872.

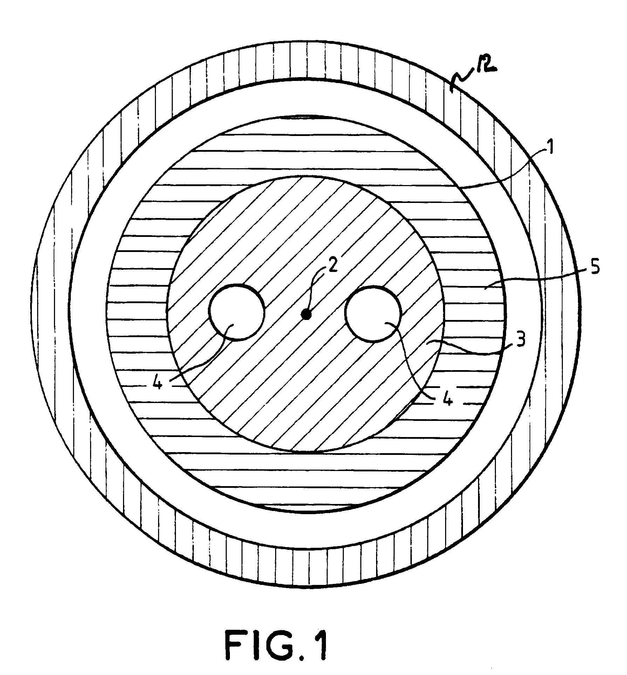

Une section transversale de cette fibre est représentée figure 1.

Cette fibre 1 comporte du centre vers l'extérieur un coeur optique 2 dont le diamètre est de 6 μm environ.

Cette fibre est entourée d'une première gaine 3 de diamètre 125 μm environ, cette gaine 3 comporte deux évidements circulaires diamétralement opposés 4 de diamètre 37 μm. La gaine 3 est elle-même située à l'intérieur d'une surcouche 5.

Dans un mode de réalisation la couche 4 est en epoxy acrylate et la surcouche 5 est en téflon.

La fibre est biréfringente à maintien de polarisation. Elle présente la particularité d'être très sensible à la pression et très peu sensible à la température. La fibre est insérée dans un tubage de téflon 12 dont le diamètre intérieur est supérieur au diamètre extérieur de la surcouche 5.

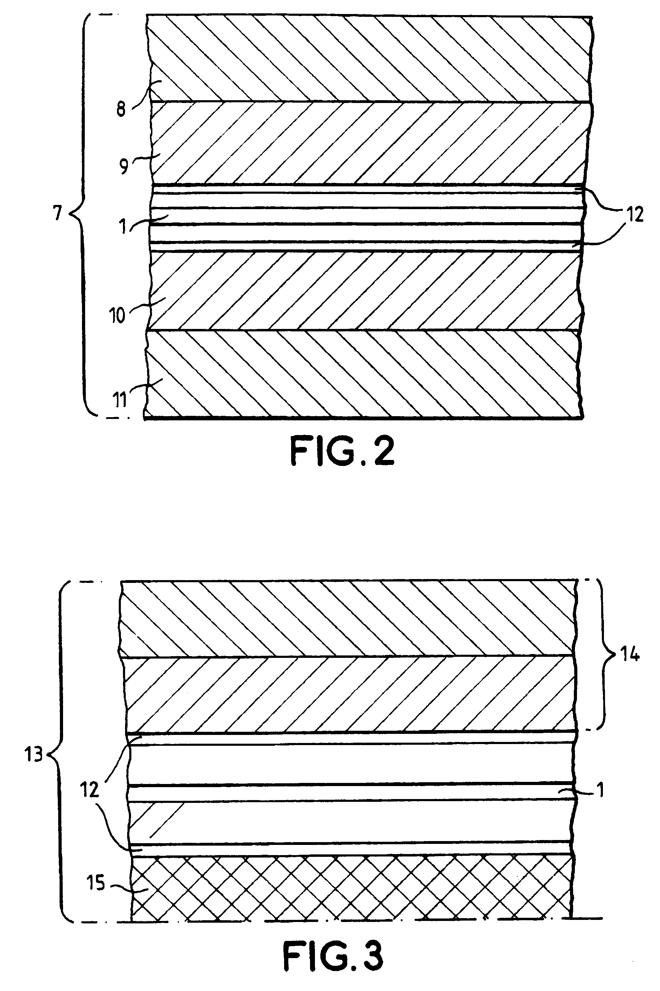

Deux modes d'insertion sont représentés figures 2 et 3.

La figure 2 représente une coupe transversale d'une partie de matériau composite 7 comportant quatre plis référencés 8 à 11. La fibre 1 est insérée entre les plis centraux 9 et 10 à l'intérieur d'un tubage 12.

La figure 3 représente une demi-coupe d'un matériau sandwich 13 dont une couche extérieure 14 est en matériau composite. Le matériau 13 comporte une âme 15 qui peut être, par exemple, une mousse durcie ou un nid d'abeilles. Le tubage 12, dans lequel est insérée la fibre 1, est logé entre l'âme 15 et le composite 14.

D'autres modes d'implantation de la fibre 1 sont possibles qui ne contreviennent pas à l'enseignement de la présente invention.

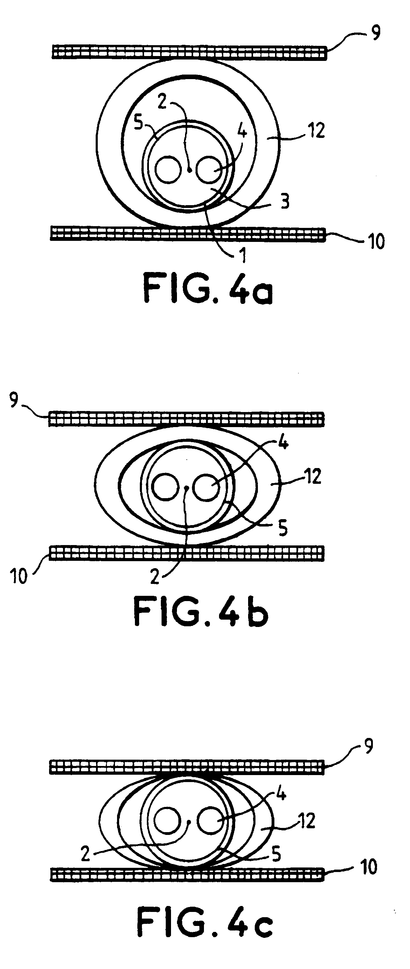

Le choix de la nature du matériau constituant le tubage 12 et de son diamètre sera fonction de la nature des déformations que l'on veut enregistrer. Des explications à ce sujet seront données ci-après en référence aux figures 4a à 4c et à la courbe 5. Les figures 4a à 4c représentent une coupe transversale de la fibre 1 et de son tubage 12 entre deux couches 9, 10 de matériau composite.

Sur la figure 4a, le matériau est soumis à une contrainte de compression qui le déforme légèrement.

Sur la figure 4b, le tubage 12 est suffisamment déformé pour que la fibre 1 soit comprimée entre deux épaisseurs du tubage. Sur la figure 4c, le tubage 12 est aplati à son maximum et ne joue plus aucun rôle d'amortissement.

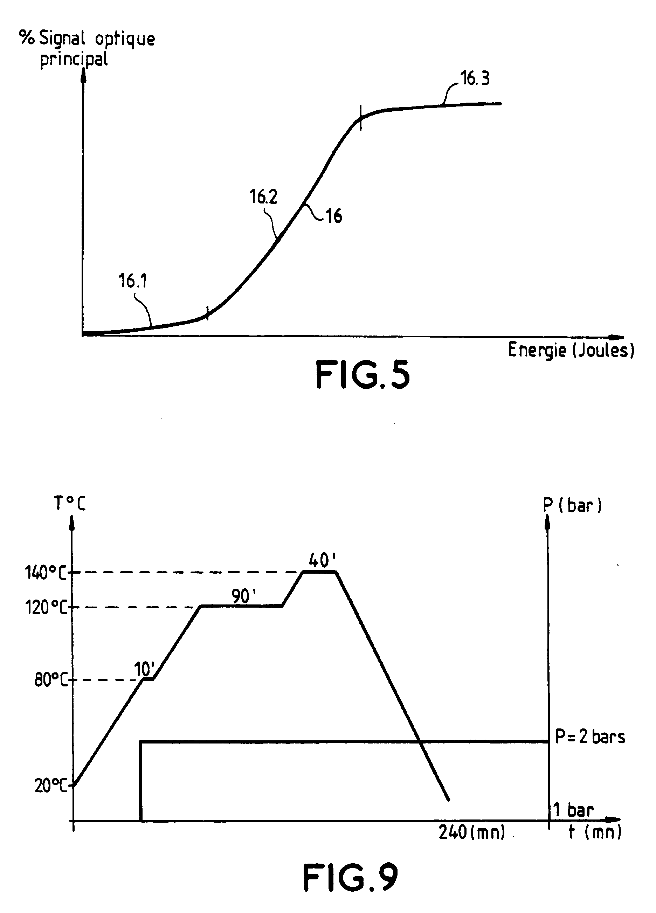

La courbe 16 de la figure 5 qui représente la réponse optique de la fibre ainsi soumise localement à ces contraintes comporte trois parties.

Cette courbe 16 représente les variations en fonction de la déformation du tubage 12 des niveaux d'un signal optique. Le niveau est donné en pourcentage du pic principal de ce signal. La nature de ce signal et la façon de l'obtenir seront données ultérieurement. Les déformations sont représentées par l'énergie en joules nécessaire pour les obtenir. Les trois zones s'analysent ainsi : dans une partie 16-1 qui correspond à la plage d'énergies qui créent des déformations qui s'inscrivent pour ce tubage 12, entre la forme de la figure 4a et la forme de la figure 4b ; la réponse est faible.

Dans la partie 16-2 qui correspond à la plage d'énergie où l'on passe de la figure 4b à la figure 4c, la réponse est linéaire et un étalonnage sur un échantillon permet une bonne reproductibilité. C'est cette partie de la courbe qui est utilisée pour détecter les dommages. C'est donc en jouant sur le module de Young du matériau choisi pour réaliser le tubage qu'il sera possible de choisir la gamme de contraintes que l'on souhaite mesurer. De plus, pour un même matériau et une même épaisseur du tubage 12, la sensibilité obtenue est plus grande lorsque le diamètre du tubage 12 diminue. Cela signifie que la pente de la partie rectiligne 16-2 est plus forte et plus à gauche. La partie 16-3 correspond à un seuil de saturation.

Si la mesure est effectuée en temps différé, il est préférable que le matériau du tubage soit inélastique dans la gamme des contraintes choisie.

La mesure effectuée correspond alors à la contrainte la plus forte. Si la mesure est effectuée en permanence en temps réel, il est préférable que le matériau du tubage soit élastique dans la gamme de contraintes choisie. Ce dernier cas est particulièrement bien adapté au cas d'un radôme.

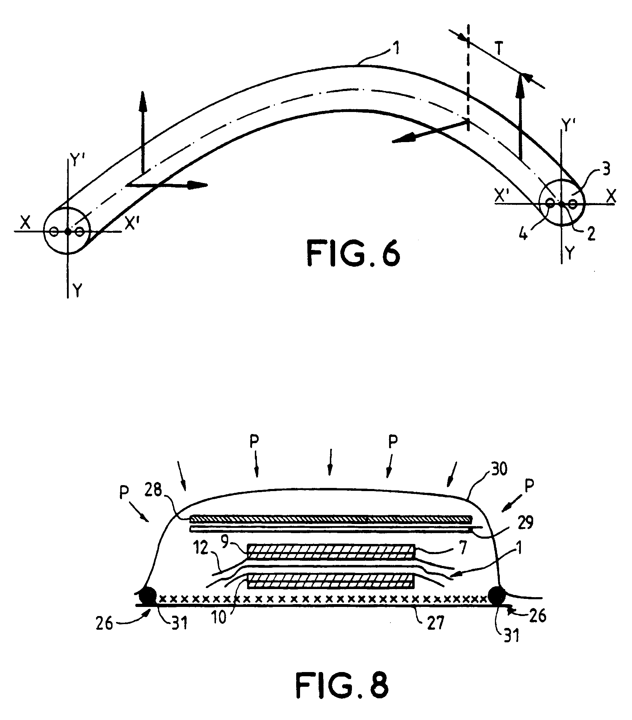

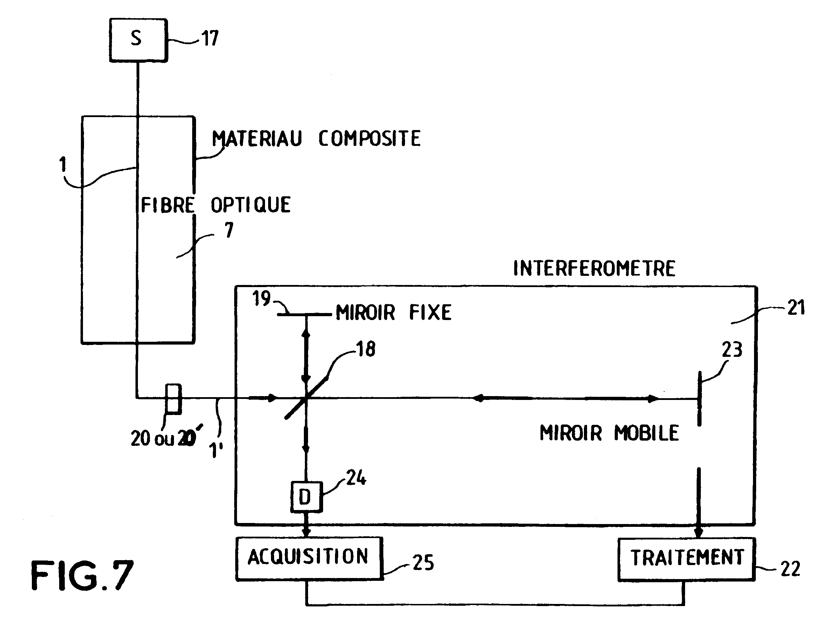

Le principe de la mesure sera maintenant explicité en référence aux figures 6 et 7 .

La figure 6 représente une vue en perspective d'une fibre biréfringente utilisée dans le dispositif de mesure selon l'invention. Dans cette fibre la lumière se propage selon deux directions orthogonales notées XX' et YY' auxquelles correspondent des constantes de propagation longitudinales $\beta x$ et $\beta y$

$$\beta x = \frac{2\pi}{\lambda} n_x \quad et \quad \beta y = \frac{2\pi}{\lambda} n_y$$

$\lambda$ est la longueur d'onde de l'onde lumineuse et $n_x$ et $n_y$ sont les indices respectifs dans les deux directions. Si $n_x$ et $n_y$ sont différents les deux ondes se propagent à des vitesses différentes. Si la fibre a une longueur L, le déphasage total entre les deux ondes est :  sont différents les deux ondes se propagent à des vitesses différentes. Si la fibre a une longueur L, le déphasage total entre les deux ondes est :

$$\theta = (\beta_x - \beta_y)L \text{ ou}$$
$$\beta_x - \beta_y = \beta$$

avec

$$\theta = \beta L$$

Sur la figure 6, les vecteurs représentent les directions des modes de propagation. L'axe XX' est l'axe qui joint les centres des évidements 4. L'axe YY' lui est perpendiculaire. T est le retard entre les deux ondes.

Si un seul mode de propagation est excité au départ d'une source lumineuse appliquée à la fibre et en l'absence de perturbations, la lumière arrive à l'autre extrémité de la fibre selon ce seul mode. Un point de contrainte sur la fibre crée un couplage entre les deux modes de propagation. Si le mode YY' est excité au départ, cela signifie qu'à partir du point de couplage une fraction de l'énergie lumineuse va se propager aussi selon le mode XX'. La valeur de cette fraction est une fonction de la valeur de la contrainte. C'est la valeur de cette fraction qui est représentée sur la courbe de la figure 5. A partir du point de couplage une onde lumineuse va se propager à une vitesse plus lente (si l'axe XX' est l'axe lent) que l'onde se propageant sur l'axe YY'.

Le retard entre les deux ondes est d'autant plus grand que la distance entre le point de couplage créé par la contrainte et le point de mesure du retard est grande.

Le principe de la mesure permet donc de connaître l'intensité de la contrainte et sa localisation.

Dans le mode de réalisation selon l'invention, la mesure est réalisée de la façon suivante décrite en référence à la figure 7.

La lumière en provenance d'une source S 17 est introduite dans la fibre de mesure 1. Il s'agit d'une onde lumineuse continue à forte cohérence spatiale et à faible cohérence temporelle. La fibre 1 traverse le matériau composite 7 selon un trajet assurant un maillage du matériau qui est une fonction de la surface minimum des délaminages que l'on veut observer. La lumière en provenance de la fibre 1 est envoyée par l'intermédiaire d'un polariseur 20 et d'une lame semi réflectrice 18 vers les deux miroirs d'un interféromètre de Michelson 21, un miroir fixe 19 et un miroir mobile 23. Le polariseur est un polariseur à 45° par rapport à l'axe des fibres. On notera que le polariseur et l'interféromètre peuvent être remplacés par tout moyen permettant de réaliser des interférences, entre un train d'ondes de référence, ici le train d'onde en permanence sur l'axe rapide, et un train d'ondes retardé par rapport à ce train de référence. Il convient donc de ramener les trains d'onde sur une seule polarisation. Un autre mode de réalisation consiste à mettre à la place du polariseur 20 une jonction 20' de fibre dont les axes lents et rapides sont croisés à 45°. La fibre 1' en sortie du polariseur ou de la jonction est de préférence longue. Le déplacement du miroir mobile 23 permet de repérer tous les déphasages pour lesquel l'onde de référence, l'onde restée continuement sur l'axe rapide, interfère avec des ondes qui, à chaque point de couplage ont commencé à voyager sur l'axe lent et qui arrivent d'autant plus en retard que le point de couplage est éloigné du point de mesure.

Un dispositif de traitement 22 permettant le déplacement du miroir mobile 23 de l'interféromètre et un dispositif de détection 24 associés à un système d'acquisition 25 permettent de façon connue de localiser les points de couplage et de déterminer l'intensité de la contrainte.

Un mode de fabrication d'un matériau composite 7 incluant la fibre 1 à l'intérieur de son tubage 12 sera maintenant décrit.

La fibre 1 dont le diamètre extérieur, compte tenu de la surcouche de téflon 5, est de 550 $\mu$m est introduite dans un tubage de téflon 12 dont le diamètre extérieur est de 1240 $\mu$m. Le tubage muni de sa fibre est alors placé entre les plis 9 et 10 du matériau 7.

Le compactage des plis préimprégnés Kevlar/Epoxy 1454 se fait au moyen d'un cycle Pression/Température en autoclave. Le dispositif est schématisé figure 8.

Le matériau 7 comprenant sa fibre optique 1 intégré dans un tubage 12 placé entre deux plis 9, 10 est posé sur une table à vide 26, imprégné d'un agent démoulant 27. Au-dessus du matériau 7 est placé un feutre 28 destiné à absorber les excès de résine. Un film 29 sépare le feutre 28 du matériau 7. L'ensemble matériau et feutre est entouré d'un sac à vide 30 relié à la table par un mastic d'étanchéité 31.

Cette installation permet de créer un vide dans la zone incluse dans le sac à vide et de mettre en pression ce qui y est contenu.

Le cycle Température/Pression est représenté figure 9.

Après une montée en température un palier de 80°C est appliqué pendant 10 minutes pour homogénéiser en température l'ensemble du matériau avant la phase de polymérisation. Pendant la phase de montée en température, un vide poussé est créé dans le sac à vide. A la fin de cette phase, une pression de 2 bars soit 3 bars compte tenu du vide est appliquée. La température est alors portée à 120°C température minimum de polymérisation de la résine choisie. Cette température est maintenue pendant 90 minutes. La température est ensuite portée à 140°C pendant 40 minutes pour assurer une stabilisation totale de la résine.

L'essentiel au cours de ce cycle est de réussir la phase de polymérisation et de stabilisation de la résine employée pour constituer le matériau sans jamais dépasser la température à laquelle la fibre ou l'un de ses revêtements commence à fondre.

Dans le cas présent, la couche la plus fragile de la fibre est la couche 3 en epoxy acrylate qui commence à être endommagée à 180°. Il convient donc de choisir la résine en fonction des matériaux constituant la fibre. Il convient que la résine incluse dans le matériau composite puisse être polymérisée et stabilisée à une température inférieure à celle qui endommagerait la couche la plus fragile de la fibre.

L'utilisation d'un tubage permet de protéger la fibre d'un risque de cisaillement au niveau des entrées et sorties de la fibre du matériau.

Lorsque, comme dans le cas de la réalisation, le tubage est en téflon et que la fibre est elle-même revêtue d'une couche extérieure en téflon, il est possible d'observer le même cycle de cuisson ou un cycle de cuisson différent, le tubage étant en place de la même façon mais la fibre n'étant pas disposée à l'intérieur du tubage avant cuisson. La fibre est alors glissée après cuisson dans le matériau. Avec ce mode de réalisation, les conditions nécessaires de température décrites ci-dessus ne sont plus nécessaires, par contre il convient de choisir le tubage et le revêtement extérieur de la fibre pour avoir un bon coefficient de glissement. Ce dernier mode de réalisation permet le remplacement rapide de la fibre en cas d'endommagement.

**Revendications**

1. Structure comportant au moins partiellement un matériau composite (7) le matériau étant réalisé au moyen de nappes de fibres noyées dans une matrice, les différentes nappes formant des plis (9, 10) du matériau, le matériau comportant des moyen (1-5, 12) intrinsèques de détection et de localisation d'une irrégularité mécanique, ces moyens comportant au moins une fibre optique biréfringente (1) à maintien de polarisation ayant un axe dit lent et un axe dit rapide noyée dans la matrice et décrivant à l'intérieur du matériau un itinéraire connu caractérisé en ce que la fibre optique (1) est incluse à l'intérieur d'un tubage (12) de diamètre intérieur supérieur au diamètre extérieur de la fibre (1).

2. Structure selon la revendication 1 caractérisée en ce que la fibre optique (1) comporte un coeur optique (2) entouré d'une première gaine comportant des évidements longitudinaux (4) disposés symétriquement par rapport au coeur optique (2).

3. Structure selon la revendication 2 caractérisée en ce que la fibre comporte en outre une surcouche (5).

4. Structure selon la revendication 3 caractérisée en ce que le matériau du tubage (12) et le matériau de la surcouche (5) sont des matériaux ayant l'un sur l'autre un bon coefficient de glissement.

5. Structure selon la revendication 4 caractérisée en ce que le matériau du tubage (12) et de la surcouche (5) est du téflon.

6. Structure selon la revendication 1 caractérisée en ce que le tubage (12) est inclus entre deux plis (9, 10) centraux du matériau (7).

7. Structure selon la revendication 1 dans laquelle le matériau composite (7) constitue une couche extérieure (14) d'un matériau sandwich (13) comportant une âme centrale (15) caractérisée en ce que le tubage (5) est placé entre le matériau composite (14) et l'âme (15) du matériau sandwich (13).

8. Procédé de fabrication du matériau composite (7), inclus dans la structure selon la revendication 1, comportant un cycle en température et pression de polymérisation et de stabilisation d'une résine constituant la matrice caractérisé en ce que la température maximum du cycle reste inférieure à une température pour laquelle l'une des couches de la fibre risque d'être endommagée.

9. Procédé selon la revendication 8 appliqué à un matériau (7) dont la fibre optique (1) comporte une couche epoxy acrylate endommageable à partir de 180°C et dont la résine polymérise à 120°C, caractérisé en ce que la température maximale du cycle est 140°C.

10. Procédé de fabrication du matériau composite (7) inclus dans la structure selon la revendication 4, caractérisé en ce que la fibre optique est incluse par glissement dans le tubage (12) après cuisson du matériau.

11. Méthode de détermination de la valeur et de la localisation d'une contrainte subie par un matériau composite, inclus dans une structure selon l'une des revendications 1 à 7, caractérisée en ce que :
   - on introduit dans la fibre un signal d'entrée constitué par une onde lumineuse continue à faible cohérence temporelle et forte cohérence spatiale ;
   - on mesure en sortie de fibre le déphasage entre le train d'onde de l'axe rapide dit signal de référence et chacun des trains d'onde successifs en provenance de l'axe lent ;

12. Méthode selon la revendication 11 dans laquelle on mesure en outre l'intensité relative entre les signaux déphasés et le signal de référence.

13. Méthode de mesure selon la revendication 11 dans laquelle le déphasage est mesuré à l'aide d'un interféromètre comportant un miroir fixe et un miroir mobile.

**Claims**

1. Structure at least partially comprising a composite material (7), the material being produced by means of sheets of fibres embedded in a matrix, the various sheets forming plies (9, 10) of the material, the material comprising intrinsic means (1-5, 12) for detection and location of a mechanical irregularity, these means comprising at least one polarization-maintaining birefringent optical fibre (1) having a so-called slow axis and a so-called fast axis, embedded in the matrix and describing a known path inside the material, characterized in that the optical fibre (1) is included inside sheathing (12) having an internal diameter greater than the external diameter of the fibre (1).

2. Structure according to Claim 1, characterized in that the optical fibre (1) comprises an optical core (2) surrounded by a first cladding comprising longitudinal holes (4) arranged symmetrically with respect to the optical core (2).

3. Structure according to Claim 2, characterized in that the fibre furthermore comprises an overlayer (5).

4. Structure according to Claim 3, characterized in that the material of the sheathing (12) and the material of the overlayer (5) are materials exhibiting a low coefficient of friction with respect to one another.

5. Structure according to Claim 4, characterized in that the material of the sheathing (12) and of the overlayer (5) is Teflon.

6. Structure according to Claim 1, characterized in that the sheathing (12) is included between two central plies (9, 10) of the material (7).

7. Structure according to Claim 1, in which the composite material (7) consists of an outer layer (14) of a sandwich material (13) comprising a central web (15), characterized in that the sheathing (5) is placed between the composite material (14) and the web (15) of the sandwich material (13).

8. Method of manufacture of the composite material (7) included in the structure according to Claim 1, comprising a temperature and pressure cycle for polymerization and stabilization of a resin constituting the matrix, characterized in that the maximum temperature of the cycle remains lower than a temperature at which one of the layers of the fibre risks being damaged.

9. Method according to Claim 8 applied to a material (7) in which the optical fibre (1) comprises an epoxy acrylate layer which can be damaged above 180°C and in which the resin polymerizes at 120°C, characterized in that the maximum temperature of the cycle is 140°C.

10. Method of manufacture of the composite material (7) included in the structure according to Claim 4, characterized in that the optical fibre is included by sliding into the sheathing (12) after curing of the material.

11. Method for determining the value and the location of a strain suffered by a composite material, included in a structure according to one of Claims 1 to 7, characterized in that:
    - an input signal consisting of a continuous light wave having low temporal coherence and high spatial coherence is introduced into the fibre;
    - the phase shift between the fast-axis wave train, called the reference signal, and each of the successive wave trains coming from the slow axis is measured at the fibre output.

12. Method according to Claim 11, in which the differential intensity between the phase-shifted signals and the reference signal is furthermore measured.

13. Measurement method according to Claim 11, in which the phase shift is measured using an interferometer comprising a stationary mirror and a moving mirror.

**Patentansprüche**

1. Struktur, die mindestens teilweise ein Verbundmaterial (7) aus in einer Matrix eingebetteten Faserschichten enthält, wobei die verschiedenen Schichten (9, 10) das Verbundmaterial bilden und das Material intrinsische Mittel zur Erfassung und Ortung einer mechanischen Unregelmäßigkeit aufweist, die mindestens eine doppelbrechende Faser (1) mit Polarisationskonstanz enthalten, wobei diese Faser eine sogenannte langsame und eine sogenannte schnelle Achse besitzt und in die Matrix eingebettet ist sowie innerhalb des Materials einen bekannten Verlauf nimmt, dadurch gekennzeichnet, daß die Lichtleitfaser (1) in einem Rohr (12) enthalten ist, dessen Innendurchmesser größer als der Außendurchmesser der Faser (1) ist.

2. Struktur nach Anspruch 1, dadurch gekennzeichnet, daß die Lichtleitfaser (1) einen optischen Kern (2) und eine erste Hülle aufweist, die in Längsrichtung Hohlräume (4) symmetrisch bezüglich des optischen Kerns (2) besitzt.

3. Struktur nach Anspruch 2, dadurch gekennzeichnet, daß die Faser außerdem eine Deckschicht (5) enthält.

4. Struktur nach Anspruch 3, dadurch gekennzeichnet, daß das Material des Rohrs (12) und das Material der Deckschicht (5) Materialien sind, die zueinander einen guten Gleitwert besitzen.

5. Struktur nach Anspruch 4, dadurch gekennzeichnet, daß das Material des Rohrs (12) und der Deckschicht (5) Teflon ist.

6. Struktur nach Anspruch 1, dadurch gekennzeichnet, daß das Rohr (12) zwischen zwei zentralen Schichten (9, 10) des Materials (7) eingeschlossen ist.

7. Struktur nach Anspruch 1, in der das Verbundmaterial (7) eine äußere Schicht (14) eines Sandwichmaterials (13) bildet, das eine zentrale Seele (15) aufweist, dadurch gekennzeichnet, daß das Rohr (5) zwischen dem Verbundmaterial (14) und der Seele (15) des Sandwichmaterials (13) angeordnet ist.

8. Verfahren zur Herstellung des Verbundmaterials (7), das zu der Struktur gemäß Anspruch 1 gehört, wobei das Verfahren einen Zyklus der Wärme- und Druckbehandlung zur Polymerisierung und Stabilisierung eines die Matrix bildenden Harzes enthält, dadurch gekennzeichnet, daß die höchste Temperatur des Zyklus unterhalb einer Temperatur bleibt, bei der eine der Schichten der Faser beschädigt werden könnte.

9. Verfahren nach Anspruch 8 in Anwendung auf ein Material (7), dessen Lichtleitfaser (1) eine ab einer Temperatur von 180°C gefährdete Schicht aus Epoxyacrylat enthält und dessen Harz bei 120°C polymerisiert, dadurch gekennzeichnet, daß die maximale Temperatur des Wärmezyklus bei 140°C liegt.

10. Verfahren zur Herstellung des in der Struktur gemäß Anspruch 4 enthaltenen Verbundmaterials (7), dadurch gekennzeichnet, daß die Lichtleitfaser nach der Wärmebehandlung des Materials gleitend in das Rohr (12) eingeführt wird.

11. Methode zur Bestimmung des Werts und des Ortes einer auf ein in einer Struktur nach einem der Ansprüche 1 bis 7 enthaltenes Verbundmaterial einwirkenden Kraft, dadurch gekennzeichnet, daß
    - man in die Faser ein von einer kontinuierlichen Lichtwelle mit geringer zeitlicher Kohärenz und hoher räumlicher Kohärenz gebildetes Eingangssignal einspeist,
    - man am Ausgang der Faser die Phasenverschiebung zwischen dem Wellenzug entlang der schnellen Achse, Bezugssignal genannt, und jedem der aufeinanderfolgenden Wellenzüge mißt, die entlang der langsamen Achse ankommen.

12. Methode nach Anspruch 11, bei der außerdem die relative Intensität zwischen den phasenverschobenen Signalen und dem Bezugssignal gemessen wird.

13. Meßmethode nach Anspruch 11, bei der die Phasenverschiebung mit Hilfe eines Interferometers gemessen wird, das einen festen und einen beweglichen Spiegel enthält.

FIG.1

FIG.2

FIG.3

FIG. 4a

FIG. 4b

FIG. 4c

FIG.5

FIG.9

FIG.6

FIG.8

14

S — 17

MATERIAU COMPOSITE

1

FIBRE OPTIQUE

7

INTERFEROMETRE

19 — MIROIR FIXE

21

18

23

20 ou 20′

1′

MIROIR MOBILE

24

D

ACQUISITION — 25

TRAITEMENT — 22

**FIG.7**

EP 0 546 899 B1